(19) Europäisches Patentamt / European Patent Office / Office européen des brevets

(11) **EP 4 530 613 A1**

(12) **EUROPÄISCHE PATENTANMELDUNG**

(43) Veröffentlichungstag:
02.04.2025 Patentblatt 2025/14

(21) Anmeldenummer: 23199686.9

(22) Anmeldetag: 26.09.2023

(51) Internationale Patentklassifikation (IPC):
**G01N 23/087** (2018.01) **A61B 6/00** (2024.01)
**G06T 11/00** (2006.01)

(52) Gemeinsame Patentklassifikation (CPC):
**G01N 23/087; A61B 6/482; G06T 11/005;**
A61B 6/5217; G01N 2223/405; G01N 2223/423;
G01N 2223/6126; G01N 2223/633

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Siemens Healthineers AG**
**91301 Forchheim (DE)**

(72) Erfinder:
• **Beister, Marcel**
**91058 Erlangen (DE)**
• **Kappler, Steffen**
**91090 Effeltrich (DE)**
• **Lück, Ferdinand**
**91052 Erlangen (DE)**
• **Ritschl, Ludwig**
**96155 Buttenheim (DE)**

(74) Vertreter: **Siemens Healthineers**
**Patent Attorneys**
**Postfach 22 16 34**
**80506 München (DE)**

(54) **VERFAHREN UND VORRICHTUNG ZUM ABSCHÄTZEN VON MATERIALDICKEN IN RADIOLOGISCHEN PROJEKTIONSBILDERN**

(57) Die Erfindung betrifft ein Verfahren zum Abschätzen von Materialdicken in radiologischen Projektionsbildern, umfassend die Schritte:
- Bereitstellen einer Mehrzahl von N Projektionsbildern bei jeweils unterschiedlichen Aufnahmeenergien,
- Durchführung einer ersten Zerlegung der N Projektionsbilder in N Dickenkarten welche jeweils die Dicke von N Bereichen mit jeweils unterschiedlichen Materialien darstellen,
- Durchführung einer zweiten Zerlegung einer Anzahl von Haupt-Dickenkarten basierend auf den N Dickenkarten und/oder auf entsprechenden Messungen und einer Anzahl der N Projektionsbilder, in N Ergebnis-Dickenkarten welche jeweils die Dicke der N Bereiche mit jeweils unterschiedlichen Materialien darstellen.

Des Weiteren umfasst die Erfindung eine Vorrichtung und ein Bildgebungsgerät.

FIG 3

EP 4 530 613 A1

**Beschreibung**

Die Erfindung betrifft ein Verfahren und eine Vorrichtung zum Abschätzen von Materialdicken in radiologischen Projektionsbildern und ein Bildgebungsgerät. Insbesondere betrifft die Erfindung eine projektionsbasierte Multi-Energie-Zerlegung.

In der spektralen Bildgebung wird häufig ein sogenannter "Dual-Energy-Ansatz" verwendet, um beispielsweise die Jod- oder Knochenkonzentration sichtbar zu machen. Mit zwei Aufnahmen bei unterschiedlichen Energien können Werte für zwei unterschiedliche Materialen, bzw. die Dicke unterschiedlicher Bereiche geschätzt werden. Beispielsweise wird bei der Mammographie davon ausgegangen, dass die Dicke der Brust konstant ist, was zur Trennung von Drüsengewebe und Jodkonzentration verwendet werden kann.

Allerdings gibt es nach einer Kompression der Brust häufig verschiedene Bereiche unterschiedlicher Dicke, z.B. den Brustrand, Hautfalten im Bereich der Achselhöhlen oder die Brust selbst, die durch eine Neigung des Paddels von der Brustwand zur Standseite entstehen. Wenn sich in einem solchen Bereich eine Jodkonzentration befindet, kann nicht unterschieden werden, ob es sich um eine Dickenänderung oder eine echte Jodkonzentration handelt. Dies stellt ein Problem dar.

In der Radiographie besteht darüber hinaus ein Ziel darin, Knochen und Wasser zu trennen. Jedoch führen Vorkommen von Fett oder anderen Materialien zu einer unvollständigen Zerlegung und hinterlassen Knochenartefakte im "Wasser"-Bild.

Derzeit wird eine kontrastmittelverstärkte Mammographie durch eine Nieder- und Hochenergieaufnahme mit einem gewichteten logarithmischen Subtraktionsalgorithmus zur Visualisierung der Jodkonzentration durchgeführt. Dies führt zu den oben geschilderten Problemen, dass Bereiche unterschiedlicher Dicke nach der logarithmischen Subtraktion zu einer inhomogenen Hintergrundintensität führen. Mehrere Nachverarbeitungsalgorithmen versuchen, diese Inhomogenität zu kompensieren und gleichzeitig die Jodkonzentrationen in diesen Regionen aufrechtzuerhalten.

In der Radiographie ist das Prinzip dasselbe, aber die unvollständige Subtraktion führt zu sichtbaren Knochenstrukturen im "Wasser"-Bild. Derzeit wird diesbezüglich keine Korrektur angewendet.

Es ist eine Aufgabe der vorliegenden Erfindung, ein Verfahren und eine Vorrichtung zum Abschätzen von Materialdicken in radiologischen Projektionsbildern und ein Bildgebungsgerät anzugeben, mit denen die oben beschriebenen Nachteile vermieden werden.

Diese Aufgabe wird durch ein Verfahren gemäß Patentanspruch 1, eine Vorrichtung gemäß Patentanspruch 11 und ein Bildgebungsgerät, gemäß Patentanspruch 13 gelöst.

Ein erfindungsgemäßes Verfahren dient zum Abschätzen von Materialdicken in radiologischen Projektionsbildern. Es umfasst die folgenden Schritte:

-   Bereitstellen (insbesondere Aufnehmen oder Laden aus einer Speichereinheit) einer Mehrzahl von N Projektionsbildern bei jeweils unterschiedlichen Aufnahmeenergien,
-   Durchführung einer ersten Zerlegung der N Projektionsbilder in N Dickenkarten welche jeweils die Dicke von N Bereichen mit jeweils unterschiedlichen Materialien darstellen,
-   Durchführung einer zweiten Zerlegung einer Anzahl von Haupt-Dickenkarten basierend auf den N Dickenkarten und/oder auf entsprechenden Messungen und einer Anzahl der N Projektionsbilder in N Ergebnis-Dickenkarten welche jeweils die Dicke der N Bereiche mit jeweils unterschiedlichen Materialien darstellen.

Das Bereitstellen von Projektionsbildern ist hinlänglich bekannt. Beispielsweise können die N Projektionsbilder mit einem Dual- oder Multi-Energy Röntgengerät aufgenommen werden und direkt mit dem Verfahren zerlegt werden. Die Projektionsbilder können aber schon früher aufgenommen und dann abgespeichert worden sein. Dann ist das Bereitstellen das Herunterladen aus einem Datenspeicher. Die Projektionsbilder können gleichzeitig oder nacheinander aufgenommen worden sein. Wichtig ist nur, dass sie dasselbe Motiv in derselben Position zeigen. Es ist theoretisch auch möglich, Bewegungsartefakte zu korrigieren, z.B. durch eine Bildregistrierung.

Im Grunde ist für das Verfahren nur wichtig, dass zwei oder mehr Projektionsbilder zur Verfügung stehen, die dieselbe Szene bei unterschiedlichen Energien zeigen. Auch wenn das Verfahren bereits bei zwei unterschiedlichen Energien vorteilhaft ist, tritt ein Vorteil bei drei oder mehr Energien noch deutlicher zutage. Für ein gutes Verständnis des Verfahrens kann man sich vorstellen, dass drei Projektionsbilder aufgenommen werden: ein H-Projektionsbild $P_H$ bei einer höchsten Aufnahmeenergie, ein M-Projektionsbild $P_M$ bei einer mittleren und ein L-Projektionsbild $P_L$ bei einer niedrigsten Aufnahmeenergie.

Es ist von Vorteil, wenn die Aufnahmeenergien so gewählt werden, dass sich die Projektionsbilder energetisch nicht überlappen (also gleiche Energien in zwei Bildern abgebildet werden) und die Energien in Bereichen liegen, in denen sich jeweils zumindest ein Material im Hinblick auf seine Abschwächung (damit ist hier immer die Abschwächung von Röntgenstrahlen gemeint) von den anderen Materialien unterscheidet. Im Hinblick auf ein Kontrastmittel kann z.B. eine Aufnahmeenergie so gewählt werden, dass sie in der K-Kante des Kontrastmittels liegt.

Im Zuge der Erfindung werden nicht nur eine, sondern zwei Zerlegungen durchgeführt, bei denen aus den Projektionsbildern (und bei der zweiten auch aus Haupt-Dickenkarten) N Dickenkarten erstellt werden. Die Dickenkarten stellen dabei jeweils die Dicke von N Bereichen mit jeweils unterschiedlichen Materialien dar. Die Dickenkarten entsprechen im Grunde den Projektionsbildern mit dem Unterschied, dass ihre "Bildpunkte" keine Abschwächungen in Form von Grautönen zeigen, sondern Dickeninformationen, z.B. in Millimetern. Zusammen ergeben die Dickenkarten in der Regel die Gesamtdicke der Brust an den entsprechenden "Bildpunkten".

Bei der ersten Zerlegung werden die N Projektionsbilder in N Dickenkarten zerlegt. Dazu kann z.B. ein P-Vektor aus den N Projektionsbildern mit einer NxN-Matrix zu den N Dickenkarten verrechnet werden. Es sollte beachtet werden, dass die Projektionsbilder aus Bildpunkte gebildet werden. Jeder Punkt an der Stelle $(x, y)$ einer Dickenkarte wird dabei aus den entsprechenden Bildpunkten an der Stelle $(x, y)$ der Projektionsbilder gebildet. Beispielsweise könnten mit der Matrix M* und dem P-Vektor $(P_1(x,y), P_2(x,y), ... , P_N(x,y))$ an den entsprechenden Koordinaten $(x,y)$ die Dickenbilder im d-Vektor $(d_1(x,y), d_2(x,y), ... , d_N(x,y))$ mit $d = M^* \cdot P$ Punkt für Punkt berechnet werden. Diese Zerlegung wird weiter unten noch genauer beschrieben.

Nach der ersten Zerlegung erfolgt die zweite Zerlegung. Im Unterschied zur ersten Zerlegung gehen nun nicht nur Projektionsbilder, sondern auch mindestens eine Haupt-Dickenkarte D in die Rechnung ein. Es wird also z.B. ein Vektor $(D, P_1+P_2, P_3,...,P_N)$ als Eingangsvektor gebildet und mit der Matrix K* multipliziert.

Die Matrizen M* und K* können einfach vorgegeben sein. Bevorzugt errechnen sie sich als inverse Matrizen aus M und K, welche aus Abschwächungskoeffizienten für die betreffenden Materialien gebildet werden. Dies wird im Folgenden noch genauer erklärt.

Die Haupt-Dickenkarte D kann direkt auf den N Dickenkarten basieren also z.B. $D = d_1 + d_2 +...+ d_N$, sie kann aber auch auf entsprechenden Messungen basieren, z.B. indem die aufgenommene Brust direkt vermessen wurde oder ein Modell zugrunde gelegt wurde, welches vermessen wird. Hier ist zu beachten, dass D im Grunde die Gesamtdicke der Brust angibt. $D(x,y)$ ist also eine Karte, welche die Gesamtdicke der Brust an den Positionen $(x,y)$ angibt.

Nun wäre es möglich, die Dickenkarte einfach zu den N Projektionsbildern hinzuzunehmen und als Eingangsvektor einen $(N+1)xN$-Vektor mit einer $Nx(N+1)$-Matrix zu verrechnen. Wie weiter unten gezeigt wird, ist es jedoch vorteilhaft, die Matrix (K*) als inverse Matrix aus einer Matrix K zu berechnen, in der Abschwächungskoeffizienten verwendet werden. Es bieten sich also quadratische Matrizen an. Es kann dazu aus den N Projektionsbildern ein Projektionsbild pro Haupt-Dickenkarte weggelassen werden, z.B. eines, welches nur einen geringen Beitrag liefert. Um jedoch mit möglichst vielen Informationen arbeiten zu können, ist es vorteilhaft, wenn zwei oder mehr Projektionsbilder zusammengefasst werden. Dies wird weiter unten noch genauer beschrieben.

Die Ergebnis-Dickenkarten können dann ausgegeben werden, z.B. direkt auf ein Display zur Auswertung oder sie können in einem Datenspeicher abgespeichert werden.

Eine erfindungsgemäße Vorrichtung dient zum Abschätzen von Materialdicken in radiologischen Projektionsbildern. Sie ist bevorzugt zur Durchführung des erfindungsgemäßen Verfahrens ausgelegt und umfasst die folgenden Komponenten:

- eine Datenschnittstelle zum Empfang einer Mehrzahl von N Projektionsbildern bei jeweils unterschiedlichen Aufnahmeenergien, bevorzugt eines H-Projektionsbildes, welches bei einer höchsten (z.B. von drei) Aufnahmeenergien aufgenommen wurde, eines M-Projektionsbildes, welches bei einer mittleren (z.B. von drei) Aufnahmeenergien aufgenommen wurde und eines L-Projektionsbildes, welches bei einer niedrigsten (z.B. von drei) Aufnahmeenergien aufgenommen wurde,
- eine Zerlegungseinheit ausgelegt für:

i) eine erste Zerlegung der N Projektionsbilder in N Dickenkarten, welche jeweils die Dicke von N Bereichen mit jeweils unterschiedlichen Materialien darstellen, und
ii) eine zweite Zerlegung einer Anzahl von Haupt-Dickenkarten basierend auf den N Dickenkarten und/oder auf entsprechenden Messungen und einer Anzahl der N Projektionsbilder, in N Ergebnis-Dickenkarten, welche jeweils die Dicke der N Bereiche mit jeweils unterschiedlichen Materialien darstellen bevorzugt zum Zerlegen von drei Projektionsbildern in drei Dickenkarten, welche jeweils die Dicke von drei unterschiedlichen Materialien darstellen.

Die Funktion der Komponenten der Vorrichtung wurden bereits im Zuge des Verfahrens beschrieben.

Ein erfindungsgemäßes Bildgebungsgerät ist bevorzugt ein Multilayer- oder Photon-Counting-Detector. Es ist zur spektralen Aufnahme eines Objekts mittels Strahlung ausgelegt und umfasst eine Strahlungsquelle ausgelegt zur Emission eines Spektrums umfassend N unterschiedliche Aufnahmeenergien eine Detektoreinheit ausgelegt zur Detektion der N Aufnahmeenergien und eine Vorrichtung gemäß der Erfindung.

Es gibt mehrere Möglichkeiten zur Aufnahme von mehreren Energien. Die Strahlungsquelle kann ein breitbandiges Spektrum emittieren, in dem alle gewünschten Aufnahmeenergien enthalten sind. Sie kann aber auch für jede Aufnahmeenergie oder für Untergruppen von Aufnahmeenergien einzelne (schmalbandige) Spektren emittieren (gleichzeitig oder nacheinander).

Auch bei den Detektoren gibt es mehrere Alternativen. Es kann z.B. ein einziger Detektor verwendet werden, der für alle Aufnahmeenergien sensitiv ist. Bei einer breitbandigen Strahlungsquelle sollten Filter verwendet werden, die für jeweils eine Aufnahme eines Projektionsbildes nur eine Aufnahmeenergie durchlassen. Alternativ kann die Strahlungsquelle sukzessive schmalbandige Spektren für jeweils eine Aufnahmeenergie emittieren.

In einem anderen Beispiel kann ein Energieselektierender Detektor verwendet werden oder mehrere Detektoren, die nur jeweils in einem schmalen Energieband aktiv sind. Hier können mehrere Aufnahmen gleichzeitig bei unterschiedlichen Aufnahmeenergien angefertigt werden.

Die Erfindung kann insbesondere in Form einer Rechnereinheit mit geeigneter Software realisiert sein. Die Rechnereinheit kann z.B. hierzu einen oder mehrere zusammenarbeitende Mikroprozessoren oder dergleichen aufweisen. Insbesondere kann sie in Form von geeigneten Softwareprogrammteilen in der Rechnereinheit realisiert sein. Eine weitgehend softwaremäßige Realisierung hat den Vorteil, dass auch schon bisher verwendete Rechnereinheiten auf einfache Weise durch ein Software- bzw. Firmware-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Rechnereinheit ladbar ist, mit Programmabschnitten, um alle Schritte des erfindungsgemäßen Verfahrens auszuführen, wenn das Programm in der Rechnereinheit ausgeführt wird. Ein solches Computerprogrammprodukt kann neben dem Computerprogramm gegebenenfalls zusätzliche Bestandteile wie z.B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Rechnereinheit und/oder zur Speicherung an oder in der Rechnereinheit kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Es ist bevorzugt, dass eine Zerlegung der Projektionsbilder P in Dickenkarten d mit einer vorgegebenen NxN-Matrix erfolgt. Die wurde oben bereits ausgeführt. Es kann z.B. für die erste Zerlegung ein P-Vektor aus den Projektionsbildern mit einer geeigneten NxN-Matrix M* multipliziert werden oder für die zweite Zerlegung ein PD-Vektor gebildet aus den Projektionsbildern und mindestens einer Haupt-Dickenkarte mit einer geeigneten NxN-Matrix K* multipliziert werden. Bevorzugt ist die Matrix (für die erste und/oder die zweite Zerlegung) eine inverse Matrix zu einer Matrix ist, welche aus Abschwächungskoeffizienten $\mu_{ij}$ der Materialien für die unterschiedlichen Energien gebildet ist.

In dem bevorzugten Fall, in dem mit (mindestens) drei Aufnahmeenergien aufgenommen wurde, wird

- ein H-Projektionsbild bereitgestellt, welches bei einer höchsten Aufnahmeenergie aufgenommen wurde,
- ein M-Projektionsbild bereitgestellt, welches bei einer mittleren Aufnahmeenergie aufgenommen wurde und
- ein L-Projektionsbild bereitgestellt, welches bei einer niedrigsten Aufnahmeenergie aufgenommen wurde.

Diese drei Projektionsbilder werden dann bei der ersten Zerlegung in drei (oder ggf. mehr) Dickenkarten zerlegt und bei der zweiten Zerlegung zusammen mit mindestens einer Haupt-Dickenkarte auch in drei (oder ggf. entsprechend mehr) Dickenkarten zerlegt, nämlich die Ergebnis-Dickenkarten.

Im Folgenden wird dies anhand eines bevorzugten Beispiels näher erläutert, bei dem mit drei unterschiedlichen Aufnahmeenergien ein H-Projektionsbild $P_H$, ein M-Projektionsbild $P_M$ und ein L-Projektionsbild $P_L$ aufgenommen worden sind. Dieses Beispiel ist mit dem dargelegten Prinzip einfach auf N Energien erweiterbar oder auf zwei Energien reduzierbar.

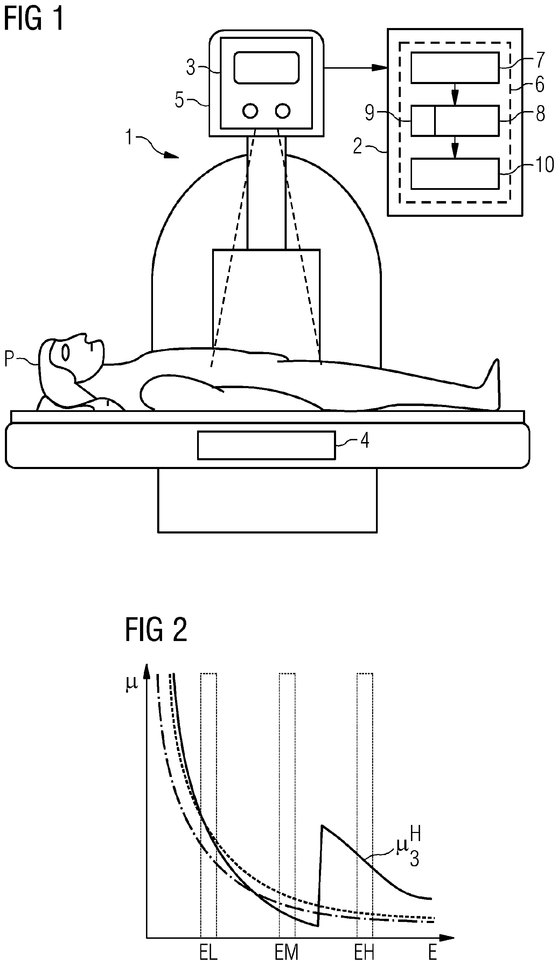

Für unterschiedliche Materialien sind die jeweiligen Abschwächungskoeffizienten $\mu$ bekannt, wie weiter unten im Zuge der Beschreibung zur Figur 2 näher ausgeführt wird. Somit sind die Abschwächungskoeffizienten $\mu_i$ (i= 1, 2, 3) für die drei Materialien bei den Energien H, M und L bekannt und können als Koeffizienten für eine Matrix M angegeben werden:

$$M = \begin{pmatrix} \mu_1^H & \mu_2^H & \mu_3^H \\ \mu_1^M & \mu_2^M & \mu_3^M \\ \mu_1^L & \mu_2^L & \mu_3^L \end{pmatrix} \qquad (1)$$

Es gilt für den P-Vektor ($P_H$, $P_M$, $P_L$) und den d-Vektor ($d_1$, $d_2$, $d_3$): P = M·d, also die jeweiligen Dicken multipliziert mit dem passenden Abschwächungskoeffizienten in der Matrix ergeben die zu erwartenden Bildwerte der Projektionsbilder. Würde man nun die Dicke der jeweiligen Materialien überall kennen (dies wären die Dickenkarten), könnte man die Projektionsbilder berechnen.

Da jedoch die Projektionsbilder bekannt sind und die Dickenkarten ermittelt werden sollen, ist es vorteilhaft, die Matrix M zur Matrix M* zu invertieren. Es folgt dann mit den Koeffizienten $m_{ij}$, die aus invertieren der Matrix M oben in (1) berechnet werden können:

$$\begin{pmatrix} d_1 \\ d_2 \\ d_3 \end{pmatrix} = \begin{pmatrix} m_{11} & m_{12} & m_{13} \\ m_{21} & m_{22} & m_{23} \\ m_{31} & m_{32} & m_{33} \end{pmatrix} \begin{pmatrix} P_H \\ P_M \\ P_L \end{pmatrix} \qquad (2)$$

Somit können dann drei Dickenkarten d1, d2, d3, aus dem H-Projektionsbild $P_H$, dem M-Projektionsbild $P_M$ und dem L-Projektionsbild $P_L$ berechnet werden, also die Projektionsbilder in Dickenkarten zerlegt werden.

Wie gesagt können zusätzlich noch weitere Projektionsbilder bereitgestellt werden, die mit weiteren Energien aufgenommen worden sind. Pro unterschiedlicher Energie kann eine Dickenkarte für ein weiteres Material erstellt werden oder die bisherigen Ergebnisse verbessert werden.

Bei der zweiten Zerlegung wird etwas anders verfahren, da dort mindestens eine Haupt-Dickenkarte verwendet wird. In dem folgenden Beispiel wird von der vorangehenden Rechnung ausgegangen und die Haupt Dickenkarte D = $d_1$ +$d_2$+$d_3$ gebildet. Es wäre auch möglich, dass Messungen der Dicke D(x,y) während der Aufnahme erfolgten oder die Haupt-Dickenkarte von einem Modell abgeleitet werden. Die eingangs beschriebene Berechnung von D ist jedoch besonders vorteilhaft, da keine anderen Informationen als die Projektionsbilder zur Verfügung stehen müssen. Da weiterhin mit drei Koeffizienten gearbeitet werden soll, werden zwei Projektionsbilder, nämlich das M-Projektionsbild und das L-Projektionsbild zu einem ML-Projektionsbild $P_{ML}$ zusammengefasst. Mit der Matrix:

$$K = \begin{pmatrix} 1 & 1 & 1 \\ \mu_1^{ML} & \mu_2^{ML} & \mu_3^{ML} \\ \mu_1^{H} & \mu_2^{H} & \mu_3^{H} \end{pmatrix} \qquad (3)$$

gilt für den PD-Vektor (D, $P_{ML}$, $P_H$) und den finalen df-Vektor ($df_1$, $df_2$, $df_3$): PD = K.df. Auch hier sollte nun zur Berechnung der finalen Dickenkarten die Matrix K zur Matrix K* invertiert werden. Es folgt dann mit den Koeffizienten $k_{ij}$, die aus der Invertierung der Matrix K oben in (3) berechnet werden:

$$\begin{pmatrix} df_1 \\ df_2 \\ df_3 \end{pmatrix} = \begin{pmatrix} k_{11} & k_{12} & k_{13} \\ k_{21} & k_{22} & k_{23} \\ k_{31} & k_{32} & k_{33} \end{pmatrix} \begin{pmatrix} D \\ P_{ML} \\ P_H \end{pmatrix} . \qquad (4)$$

Es ist bevorzugt, dass bei einer Ausführungsform des Verfahrens eine der Dickenkarten

- eine K-Dickenkarte ist, welche die Dicke von Kontrastmittelbereichen darstellt und/oder
- eine G-Dickenkarte ist, welche die Dicke von Drüsengewebe darstellt und/oder
- eine A-Dickenkarte ist, welche die Dicke von Fettgewebe darstellt und/oder
- eine B-Dickenkarte ist, welche die Dicke von Knochen darstellt, und/oder
- eine W-Dickenkarte ist, welche die Dicke von Bereichen mit Wasser darstellt.

Bevorzugt, insbesondere für ein Verfahren mit drei Aufnahmeenergien, ist eine von drei Dickenkarten eine K-Dickenkarte, eine weitere eine G-Dickenkarte, und eine weitere eine A-Dickenkarte.

Bevorzugt basieren die Aufnahmeenergien auf den Abschwächungseigenschaften derjenigen Materialien, die den Dickenkarten zugrundeliegen. Wie oben gesagt wurde, sollten die Aufnahmeenergien aus Energiebereiche gewählt werden, in denen sich der Abschwächungskoeffizienten zumindest eines der Materialien von den Anderen stark unterscheidet. Bevorzugt werden Strahlenergien gewählt, an denen Unterschiede der Abschwächungseigenschaften aller Materialien oder zumindest eines Materials zu den jeweils anderen maximal sind. Bevorzugt wurde eine Aufnahmeenergie (bei der Aufnahme der Projektionsbilder) so gewählt, dass sie im abfallenden Teil einer Absorptionskante eines der Materialien liegt.

Die niedrigste Aufnahmeenergie liegt bevorzugt zwischen 10 keV und 100 keV. Eine mittlere Aufnahmeenergie liegt bevorzugt zwischen 20 keV und 130 keV. Die höchste Aufnahmeenergie liegt bevorzugt zwischen 30 keV und 150 keV. Die Aufnahmeenergien hängt jedoch sehr stark von den Materialien ab, nach denen zerlegt werden soll. Es sollte darauf geachtet werden, dass insgesamt Energien gewählt werden, die eine gute energetische Trennung der gewünschten Materialien ermöglichen.

Gemäß einem bevorzugten Verfahren wird die erste Zerlegung der Projektionsbilder in Dickenkarten mehrfach durchgeführt und die Dickenkarten werden zur Korrektur der Strahlaufhärtung der zerlegten Projektionsbilder und/oder von Koeffizienten einer bei der Zerlegung verwendeten Matrix verwendet. Diese mehrfache Durchführung der ersten Zerlegung und Korrektur erfolgt vor der zweiten Zerlegung, die nur ein Mal durchgeführt werden muss, jedoch auch mehrfach für eine entsprechende Korrektur durchgeführt werden kann.

Es ist hier zu beachten, dass mit unterschiedlichen Aufnahmeenergien gearbeitet wurde und unterschiedliche Materialien bei unterschiedlichen Energien unterschiedlich absorbieren. Dies wird mit der Korrektur der Strahlaufhärtung kompensiert. Im Grunde wird aus einer polyenergetischen Abschwächung eine (pseudo) monoenergetische Abschwächung berechnet.

Bevorzugt wird dabei eine polynomische Korrektur angewandt. Ein korrigiertes Projektionsbild $P_{korr}(x,y)$ kann dabei aus einem Projektionsbild $P(x,y)$ mittels durch Testmessungen ermittelbaren oder berechenbaren Koeffizienten $c_a$ für jeden Bildpunkt $(x,y)$ und einem vorher festgelegten Grad A des Polynoms eine Korrektur nach der folgenden Formel durchgeführt werden:

$$P_{korr}(x,y) = \sum_{a}^{A} c_a P^a(x,y) , \qquad (5)$$

also im Grunde das Polynom $c_0 + c_1 P + c_2 P^2 + ... + c_N P^N$. Hierzu ist anzumerken, dass die Koeffizienten c im Grunde auch von den Dickenkarten abhängen, also davon, durch welche Dicke der Materialien ein Strahl hindurchgehend musste, bevor er auf einem Bildpunkt des Detektors traf. Es gilt also, dass die $c_a$ im Grunde von $(d_1, d_2, ..., d_N)$ für jeden Bildpunkt abhängig sind. Hier kann es durchaus sein, dass diese Abhängigkeit für einige Materialien vernachlässigbar ist. Werden z.B. glandulare, adipöse und Jod-Dickenkarten erzeugt, so hat sich gezeigt, dass eine Abhängigkeit von der Haupt Dickenkarte D (Addition der drei Dickenkarten) und der glandularen G-Dickenkarte, ggf. zusammen mit der Jod-Dickenkarte, ausreichend ist. Es würde also dann bevorzugt von einem $c_a(d_g/D,D)$ bzw. $c_a(d_j/D,d_g/D,D)$ ausgegangen. Diese können für unterschiedliche $d_g$, $d_j$ und D berechnet oder gemessen werden

Hierzu sollte beachtet werden, dass für die zu Beginn des Verfahrens aufgenommenen Bilder ("initiale Bilder") noch keine Dickenkarten vorhanden sind. Es könnte hier zunächst auf eine Korrektur der Strahlaufhärtung verzichtet werden und erst bei der nächsten Iteration eine Korrektur durchgeführt werden. Bevorzugt wird jedoch auch eine Korrektur der initialen Bilder durchgeführt und zwar mit vorgegebenen Dickenkarten. Dazu wird bei einer Brustaufnahme insbesondere ein einheitlicher Drüsengewebsanteil von 50% auf das ganze Bild angewendet.

Wie vorangehend angedeutet ist bevorzugt, dass bei einer Ausführungsform des Verfahrens eine Haupt-Dickenkarte D durch Addition der (z.B. drei) Dickenkarten gebildet wird. Diese wird bevorzugt für eine Korrektur der Strahlaufhärtung der zerlegten Projektionsbilder und/oder für eine Bildung von finalen Dickenkarten verwendet.

Es ist bevorzugt, dass die erste Zerlegung mit den einzelnen N Projektionsbildern durchgeführt wird und die zweite Zerlegung mit weniger als N Projektionsbildern. Dann kann insbesondere jeweils mit NxN-Matrizen gearbeitet werden.

Bevorzugt werden für die zweite Zerlegung eine Mehrzahl, insbesondere zwei, der Projektionsbilder zu einem vereinigten V-Projektionsbild zusammengefasst z.B. zu dem oben beschriebenen ML-Projektionsbild $P_{ML}$.

Bevorzugt wird für die zweite Zerlegung eine Haupt-Dickenkarte aus einer Addition der N Dickenkarten der ersten Zerlegung verwendet. Alternativ oder zusätzlich kann für die zweite Zerlegung eine Haupt-Dickenkarte aus einem Modell des aufgenommenen Objekts verwendet werden. Alternativ oder zusätzlich kann für die zweite Zerlegung eine Haupt-Dickenkarte aus Messungen des aufgenommenen Objekts verwendet werden.

Es ist bevorzugt, dass bei einer Ausführungsform des Verfahrens energetisch benachbarte Projektionsbilder zu einem V-Projektionsbild zusammengefasst werden, dies ist jedoch nicht zwingend notwendig. "Energetisch benachbart" meint dabei, dass die Aufnahmeenergien direkt nebeneinander liegen. In dem bevorzugten Fall von drei Aufnahmeenergien wird das M-Projektionsbild $P_M$ und das L-Projektionsbild $P_L$ zu einem ML-Projektionsbild $P_{ML}$ zusammengefasst.

Diese Zusammenfassung erfolgt bevorzugt mittels einer gewichteten Mittelung der Bildwerte in Abhängigkeit von einer Intensität einer Bestrahlung mit der jeweiligen Aufnahmeenergie. Bevorzugt werden dazu Bildwerte an denselben Stellen miteinander addiert und dann mittels einer Summe der jeweiligen Intensitäten normiert. Im Grunde ist eine solche gewichtete Mittelung Stand der Technik.

Es ist bevorzugt, dass bei einer Ausführungsform des Verfahrens für eine Matrix zur zweiten Zerlegung zusätzlich diejenigen Abschwächungskoeffizienten zusammengefasst werden, die den Aufnahmeenergien der zusammengefassten Projektionsbilder entsprechen, bevorzugt mittels einer gewichteten Mittelung. Wie oben ausgeführt wurde, können bei drei Aufnahmeenergien und der Zusammenfassung des M-Projektionsbildes und des L-Projektionsbildes zu einem ML-Projektionsbild die Koeffizienten $\mu^M$ und $\mu^L$ zu $\mu^{ML}$ zusammengefasst werden. Bevorzugt werden dabei bei drei Aufnahmeenergien die Koeffizienten $\mu^{ML}$ aus einer gewichteten Mittelung der Koeffizienten $\mu^M$ und $\mu^L$ für das jeweilige Material in Abhängigkeit von der Intensität einer Bestrahlung mit der jeweiligen Aufnahmeenergie berechnet.

Es ist bevorzugt, dass bei einer Ausführungsform des Verfahrens eine Anzahl von Dickenkarten und/oder eine Haupt-Dickenkarte zusätzlich entrauscht werden. Dies erfolgt bevorzugt mittels eines Verfahrens, welches eine variable Dicke eines aufgenommenen Objekts berücksichtigt und insbesondere von einer kontinuierlichen Dickenänderung ausgeht. Die Entrauschung findet dabei bevorzugt nur im Bereich des (aufgenommenen) Objekts statt.

Eine bevorzugte Vorrichtung umfasst eine Korrektureinheit ausgelegt zur Korrektur von Projektionsbildern und/oder Abschwächungskoeffizienten, insbesondere der Korrektur der Strahlaufhärtung.

Eine bevorzugte Vorrichtung umfasst eine Entrauschungseinheit ausgelegt zur Entrauschung von Dickenkarten.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1 ein Radiographie-System mit einer erfindungsgemäßen Vorrichtung,

Figur 2 einen Graph mit Abschwächungen und Aufnahmeenergien,

Figur 3 ein Beispiel für ein Verfahren zum Abschätzen von Materialdicken in radiologischen Projektionsbildern gemäß der Erfindung.

Figur 1 zeigt grob schematisch ein Bildgebungsgerät 1 in Form eines Röntgensystems 1 mit einer Steuereinrichtung 2. Diese ist mit einer Vorrichtung 6 ausgelegt zur Durchführung des erfindungsgemäßen Verfahrens ausgestattet. Das Röntgensystem 1 weist in üblicher Weise eine Strahlungsquelle 3 auf, welche hier eine Röntgenquelle darstellt, und während einer Aufnahme eines Projektionsbildes einen Patienten P mit einem durch den Kollimator 5 kollimierten Strahl durchstrahlt, so dass die Strahlung auf einen der Strahlungsquelle 3 gegenüberliegenden Detektor 4 trifft.

Bei der Steuereinrichtung 2 sind nur die Komponenten dargestellt, die für die Erläuterung der Erfindung wesentlich sind. Grundsätzlich sind Radiographie-Systeme 1 und zugehörige Steuereinrichtungen 2 dem Fachmann bekannt und brauchen daher nicht im Detail erläutert zu werden.

Die Steuereinrichtung 2 umfasst eine Vorrichtung 6 zum Abschätzen von Materialdicken in radiologischen Projektionsbildern. Die Vorrichtung 6 umfasst eine Datenschnittstelle 7, eine Zerlegungseinheit 8, eine Korrektureinheit 9 und eine Entrauschungseinheit 10.

Die Datenschnittstelle 7 dient zum Empfang einer Mehrzahl von N Projektionsbildern bei jeweils unterschiedlichen Aufnahmeenergien, bevorzugt eines H-Projektionsbildes, welches bei einer höchsten von drei Aufnahmeenergien aufgenommen wurde, eines M-Projektionsbildes, welches bei einer mittleren von drei Aufnahmeenergien aufgenommen wurde und eines L-Projektionsbildes, welches bei einer niedrigsten von drei Aufnahmeenergien aufgenommen wurde (s. auch Figur 4).

Die Zerlegungseinheit 8 dient zur Durchführung von zwei aufeinanderfolgenden Zerlegungen, nämlich einer ersten Zerlegung der N Projektionsbilder in N Dickenkarten welche jeweils die Dicke von N Bereichen mit jeweils unterschiedlichen Materialien darstellen, und einer zweiten Zerlegung einer Anzahl von Haupt-Dickenkarten basierend auf den N Dickenkarten und/oder auf entsprechenden Messungen und einer Anzahl der N Projektionsbilder, in N Ergebnis-Dickenkarten welche jeweils die Dicke der N Bereiche mit jeweils unterschiedlichen Materialien darstellen.

Die Korrektureinheit 9 ist zur Korrektur von Projektionsbildern ausgelegt. Die erste Zerlegung der Projektionsbilder in Dickenkarten kann dabei mehrfach durchgeführt werden und die Dickenkarten werden zur Korrektur der Strahlaufhärtung der zerlegten Projektionsbilder verwendet. Dies kann iterativ erfolgen, dass die Korrektur mit den jeweils aktuell ermittelten Dickenkarten, die auf der Zerlegung verbesserter Projektionsbilder beruhen, verbessert wird.

Die Entrauschungseinheit 10 ist zur Entrauschung von Dickenkarten ausgelegt. Dies können die Dickenkarten sein, die durch die erste Zerlegung erhalten werden oder eine Haupt-Dickenkarte sein. Die Entrauschung erfolgt bevorzugt mittels eine Verfahrens, welches eine variable Dicke eines aufgenommenen Objekts berücksichtigt und insbesondere von einer kontinuierlichen Dickenänderung ausgeht, wobei die Entrauschung bevorzugt nur im Bereich des Objekts stattfindet.

Figur 2 zeigt einen Graph mit Abschwächungen für Röntgenstrahlen und drei Aufnahmeenergien EH, EM, EL. Der Graph mit der durchgezogenen Linie zeigt z.B. die Abschwächung von Jod, der Graph mit der gestrichelten Line die glandulare und der strichpunktierte Graph die adipöse Abschwächung. Deutlich ist die K-Kante bei der Abschwächung von Jod zu erkennen. Es werden nun Projektionsbilder bei drei (engen) Aufnahmeenergien EH, EM, EL aufgenommen. Bei der niedrigsten Aufnahmeenergie EL sind die Abschwächungen von Jod und die glandulare Abschwächung fast gleich und es unterscheidet sich die adipöse Abschwächung von den beiden. Bei der mittleren Aufnahmeenergie EH liegen die drei Abschwächungen deutlich auseinander. Die höchste Aufnahmeenergie EH liegt in der K-Kante von Jod. Dort unterscheidet sich also die Abschwächung von Jod deutlich von den beiden anderen.

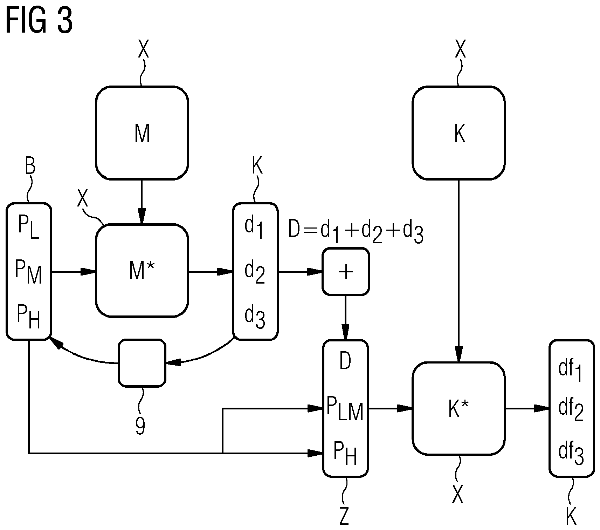

Wenn Jod als Material 3 gewählt würde, könnte man z.B. für eine Matrix zur Zerlegung den Abschwächungskoeffizienten $\mu$ genau dort aus dem Graphen von Jod wählen. In diesem Beispiel ist angedeutet, dass man im Hinblick auf Figur 4 (mit Jod als drittes Material) dort den Koeffizienten $\mu_3^H$ wählen kann. Figur 3 zeigt ein Verfahren zum Abschätzen von Materialdicken in radiologischen Projektionsbildern B. Ganz links wird mit dem P-Vektor ($P_L$, $P_M$, $P_H$) begonnen. dies sind drei Projektionsbilder B mit Bildwerten an den Koordinaten (x,y), die bereitgestellt worden sind. Im Folgenden wird zwar von den Bildern B gesprochen, man kann sich jedoch vorstellen, dass alle Berechnungen mit deren Bildpunkten durchgeführt werden.

$P_H$ bezeichnet ein H-Projektionsbild, welches bei einer höchsten von drei Aufnahmeenergien aufgenommen wurde, $P_M$ eine M-Projektionsbild, welches bei einer mittleren Aufnahmeenergie aufgenommen wurde und $P_L$ ein L-Projektionsbild, welches bei einer niedrigsten Aufnahmeenergie aufgenommen wurde.

Aus Abschwächungskoeffizienten $\mu$, die aus der Figur 2 entnommen wurden, wird nun die M-Matrix X erstellt und gleichzeitig die K-Matrix X mit einer (gewichteten) Zusammenfassung der Koeffizienten $\mu^M$ und $\mu^L$ zu $\mu^{ML}$. Die beiden Matrizen X sind dann:

$$M = \begin{pmatrix} \mu_1^H & \mu_2^H & \mu_3^H \\ \mu_1^M & \mu_2^M & \mu_3^M \\ \mu_1^L & \mu_2^L & \mu_3^L \end{pmatrix} \quad \text{und} \quad K = \begin{pmatrix} 1 & 1 & 1 \\ \mu_1^{ML} & \mu_2^{ML} & \mu_3^{ML} \\ \mu_1^H & \mu_2^H & \mu_3^H \end{pmatrix}.$$

Diese beiden Matrizen X werden zu den Matrizen M* bzw. K* invertiert und zunächst aus dem P-Vektor und der Matrix M* mit d = M* -P die Karten K (der d-Vektor ($d_1$, $d_2$, $d_3$)) berechnet.

Die Dickenkarten ($d_1$, $d_2$, $d_3$) des d-Vektors kann man nun verwenden, um die Projektionsbilder zu korrigieren, ggf. mit einer Haupt-Dickenkarte D = $d_1$+$d_2$+$d_3$. Dies erfolgt z.B. mit der Formel $P_{korr} = c_0(d_2/D)+c_1(d_2/D)P+c_2(d_2/D)P^2+c_3(d_2/D)P^3$. Die korrigierten Werte $P_{korr}$ sind dann die neuen Werte für den P-Vektor, aus dem erneut die Karten K ermittelt werden.

Mit Zwischenwerten Z, nämlich der Haupt-Dickenkarte D, dem H-Projektionsbild $P_H$ und einem V-Projektionsbild $P_{ML}$, das eine Zusammenfassung des L-Projektionsbildes $P_L$ und des M-Projektionsbildes $P_M$ ist, wird nun die zweite Zerlegung durchgeführt. Der mit den Zwischenwerten Z angedeutete PD-Vektor wird nun mit der Matrix K* multipliziert und es ergeben sich mit df = K*·PD die finalen Dickenkarten $df_1$, $df_2$ und $df_3$ als Karten K.

Es wird abschließend noch einmal darauf hingewiesen, dass es sich bei der vorhergehend detailliert beschriebenen Erfindung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen Begriffe wie "Einheit" nicht aus, dass die betreffenden Komponenten aus mehreren zusammenwirkenden Teil-Komponenten bestehen, die ggf. auch räumlich verteilt sein können. Der Begriff "eine Anzahl" ist als "mindestens ein(e)" zu lesen. Unabhängig vom grammatikalischen Geschlecht eines bestimmten Begriffes sind Personen mit männlicher, weiblicher oder anderer Geschlechteridentität mit umfasst.

**Patentansprüche**

**1.** Verfahren zum Abschätzen von Materialdicken in radiologischen Projektionsbildern, umfassend die Schritte:

- Bereitstellen einer Mehrzahl von N Projektionsbildern bei jeweils unterschiedlichen Aufnahmeenergien,
- Durchführung einer ersten Zerlegung der N Projektionsbilder in N Dickenkarten welche jeweils die Dicke von N Bereichen mit jeweils unterschiedlichen Materialien darstellen,
- Durchführung einer zweiten Zerlegung einer Anzahl von Haupt-Dickenkarten basierend auf den N Dickenkarten und/oder auf entsprechenden Messungen und einer Anzahl der N Projektionsbilder, in N Ergebnis-Dickenkarten welche jeweils die Dicke der N Bereiche mit jeweils unterschiedlichen Materialien darstellen.

**2.** Verfahren nach Anspruch 1, wobei eine Zerlegung der Projektionsbilder P in Dickenkarten d mit einer vorgegebenen NxN-Matrix (X) erfolgt, bevorzugt wobei die Matrix (X) eine inverse Matrix (X) zu einer Matrix (X) ist, welche aus

Abschwächungskoeffizienten (u) $\mu_{ij}$ der Materialien für die unterschiedlichen Energien (E) gebildet ist, bevorzugt wobei

- ein H-Projektionsbild bereitgestellt wird, welches bei einer höchsten von drei Aufnahmeenergien aufgenommen wurde,
- ein M-Projektionsbild bereitgestellt wird, welches bei einer mittleren von drei Aufnahmeenergien aufgenommen wurde und
- ein L-Projektionsbild bereitgestellt wird, welches bei einer niedrigsten von drei Aufnahmeenergien aufgenommen wurde, und diese drei Projektionsbilder in drei Dickenkarten zerlegt werden.

3.  Verfahren nach Anspruch 1 oder 2, wobei eine der Dickenkarten

- eine K-Dickenkarte ist, welche die Dicke von Kontrastmittelbereichen darstellt und/oder
- eine G-Dickenkarte ist, welche die Dicke von Drüsengewebe darstellt und/oder
- eine A-Dickenkarte ist, welche die Dicke von Fettgewebe darstellt und/oder
- eine B-Dickenkarte ist, welche die Dicke von Knochen darstellt, und/oder
- eine W-Dickenkarte ist, welche die Dicke von Bereichen mit Wasser darstellt,

bevorzugt wobei die drei Dickenkarten bevorzugt eine K-Dickenkarte, eine G-Dickenkarte, und eine A-Dickenkarte sind.

4.  Verfahren nach einem der vorangehenden Ansprüche, wobei die Aufnahmeenergien auf den Abschwächungseigenschaften derjenigen Materialien basieren, die den Dickenkarten zugrundeliegen, bevorzugt wobei Strahlenergien gewählt werden, an denen Unterschiede der Abschwächungseigenschaften aller Materialien oder zumindest eines Materials zu den anderen maximal sind, Bevorzugt wobei zumindest eine Aufnahmeenergie so gewählt wurde, dass sie im abfallenden Teil einer Absorptionskante eines der Materialien liegt, bevorzugt wobei eine niedrigste Aufnahmeenergie zwischen 10 keV und 100 keV liegt, eine mittlere Aufnahmeenergie zwischen 20 keV und 130 keV liegt und eine höchste Aufnahmeenergie zwischen 30 keV und 150 keV liegt.

5.  Verfahren nach einem der vorangehenden Ansprüche, wobei die erste Zerlegung der Projektionsbilder in Dickenkarten mehrfach durchgeführt wird und die Dickenkarten zur Korrektur der Strahlaufhärtung der zerlegten Projektionsbilder und/oder von Koeffizienten einer bei der Zerlegung verwendeten Matrix (X) verwendet werden, wobei bevorzugt eine polynomische Korrektur angewandt wird.

6.  Verfahren nach einem der vorangehenden Ansprüche, wobei eine Haupt-Dickenkarte D durch Addition der Dickenkarten gebildet wird und diese bevorzugt für eine Korrektur der Strahlaufhärtung der zerlegten Projektionsbilder und/oder für eine Bildung von finalen Dickenkarten verwendet wird.

7.  Verfahren nach einem der vorangehenden Ansprüche, wobei die erste Zerlegung mit den einzelnen N Projektionsbildern durchgeführt wird und die zweite Zerlegung mit weniger als N Projektionsbildern, wobei bevorzugt für die zweite Zerlegung eine Mehrzahl, insbesondere zwei, dieser Projektionsbilder zu einem vereinigten V-Projektionsbild zusammengefasst werden, bevorzugt wobei für die zweite Zerlegung eine Haupt-Dickenkarte

- aus einer Addition der N Dickenkarten der ersten Zerlegung und/oder
- aus einem Modell des aufgenommenen Objekts und/oder
- aus Messungen des aufgenommenen Objekts

verwendet wird.

8.  Verfahren nach Anspruch 7, wobei energetisch benachbarte Projektionsbilder zu einem V-Projektionsbild zusammengefasst werden, insbesondere bei drei Aufnahmeenergien ein M-Projektionsbild und ein L-Projektionsbild zu einem ML-Projektionsbild, bevorzugt mittels einer gewichteten Mittelung der Bildwerte in Abhängigkeit von einer Intensität (I) einer Bestrahlung mit der jeweiligen Aufnahmeenergie, bevorzugt wobei Bildwerte an denselben Stellen miteinander addiert und dann mittels einer Summe der jeweiligen Intensitäten (I) normiert werden oder wobei mit den jeweiligen Intensitäten (I) normierte Bildwerte addiert werden.

9.  Verfahren nach Anspruch 8, wobei für eine Matrix (X) zur zweiten Zerlegung zusätzlich diejenigen Abschwächungskoeffizienten (u) zusammengefasst werden, die den Aufnahmeenergien der zusammengefassten Projektionsbilder entsprechen, bevorzugt mittels einer gewichteten Mittelung, bevorzugt wobei bei drei Aufnahmeenergien die Koeffizienten $\mu ML$ aus einer gewichteten Mittelung der Koeffizienten $\mu M$ und $\mu L$ für das jeweilige Material in Abhängigkeit von der Intensität (I) einer Bestrahlung mit der jeweiligen Aufnahmeenergie berechnet werden.

10. Verfahren nach einem der vorangehenden Ansprüche, wobei eine Anzahl von Dickenkarten und/oder eine Haupt-Dickenkarte zusätzlich entrauscht werden, bevorzugt mittels eine Verfahrens, welches eine variable Dicke eines aufgenommenen Objekts berücksichtigt und insbesondere von einer kontinuierlichen Dickenänderung ausgeht, wobei die Entrauschung bevorzugt nur im Bereich des Objekts stattfindet.

11. Vorrichtung (6) zum Abschätzen von Materialdicken in radiologischen Projektionsbildern, umfassend:

- eine Datenschnittstelle (7) zum Empfang einer Mehrzahl von N Projektionsbildern bei jeweils unterschiedlichen Aufnahmeenergien, bevorzugt eines H-Projektionsbildes, welches bei einer höchsten von drei Aufnahmeenergien aufgenommen wurde, eines M-Projektionsbildes, welches bei einer mittleren von drei Aufnahmeenergien aufgenommen wurde und eines L-Projektionsbildes, welches bei einer niedrigsten von drei Aufnahmeenergien aufgenommen wurde,
- eine Zerlegungseinheit (8) ausgelegt für:
- eine erste Zerlegung der N Projektionsbilder in N Dickenkarten, welche jeweils die Dicke von N Bereichen mit jeweils unterschiedlichen Materialien darstellen, und
- eine zweite Zerlegung einer Anzahl von Haupt-Dickenkarten basierend auf den N Dickenkarten und/oder auf entsprechenden Messungen und einer Anzahl der N Projektionsbilder, in N Ergebnis-Dickenkarten, welche jeweils die Dicke der N Bereiche mit jeweils unterschiedlichen Materialien darstellen bevorzugt zum Zerlegen von drei Projektionsbildern in drei Dickenkarten, welche jeweils die Dicke von drei unterschiedlichen Materialien darstellen.

12. Vorrichtung (6) nach Anspruch 11, umfassend

- eine Korrektureinheit (9) ausgelegt zur Korrektur von Projektionsbildern und/oder Abschwächungskoeffizienten (u), und/oder
- eine Entrauschungseinheit (10) ausgelegt zur Entrauschung von Dickenkarten.

13. Bildgebungsgerät (1), bevorzugt ein Multilayer- oder Photon-Counting-Detector, ausgelegt zur spektralen Aufnahme eines Objekts mittels Strahlung umfassend eine Strahlungsquelle (3) ausgelegt zur Emission eines Spektrums umfassend N unterschiedliche Aufnahmeenergien (EH, EM, EL), eine Detektoreinheit (4) ausgelegt zur Detektion der N Aufnahmeenergien (EH, EM, EL) und eine Vorrichtung (6) nach einem der Ansprüche 11 oder 12.

14. Computerprogrammprodukt, umfassend Befehle, die bei der Ausführung des Programms durch einen Computer diesen veranlassen, die Schritte des Verfahrens nach Anspruch 1 bis 10 auszuführen.

15. Computerlesbares Speichermedium, umfassend Befehle, die bei der Ausführung durch einen Computer diesen veranlassen, die Schritte des Verfahrens nach Anspruch 1 bis 10 auszuführen.

FIG 1

FIG 2

FIG 3

Europäisches
Patentamt

European
Patent Office

Office européen
des brevets

# EUROPÄISCHER RECHERCHENBERICHT

Nummer der Anmeldung

EP 23 19 9686

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2022/167935 A1 (IWASHITA ATSUSHI [JP] ET AL) 2. Juni 2022 (2022-06-02) * Zusammenfassung; Ansprüche 1,4 * * Seite 6 * * Absätze [0066], [0124] * ----- | 1-15 | INV. G01N23/087 A61B6/00 G06T11/00 |
| X | CN 116 392 153 A (NEUSOFT MEDICAL SYSTEMS CO LTD) 7. Juli 2023 (2023-07-07) * Absätze [0064] - [0107] * ----- | 1,2,6, 11,13-15 | |
| X | SCHAEFER JAMIN ET AL: "Simulation Study of Material Decomposition in Dual-Energy Radiography for Bone Removal", 2022 IEEE NUCLEAR SCIENCE SYMPOSIUM AND MEDICAL IMAGING CONFERENCE (NSS/MIC), IEEE, 5. November 2022 (2022-11-05), Seiten 1-7, XP034528461, DOI: 10.1109/NSS/MIC44845.2022.10398895 [gefunden am 2024-01-26] * das ganze Dokument * ----- | 1,2,6, 11,13-15 | |
| X | US 2022/366543 A1 (IWASHITA ATSUSHI [JP] ET AL) 17. November 2022 (2022-11-17) * Absätze [0115], [0118]; Anspruch 4 * ----- | 1,2,6,7, 11,13-15 | RECHERCHIERTE SACHGEBIETE (IPC) G01N A61B G06T |

Der vorliegende Recherchenbericht wurde für alle Patentansprüche erstellt

| Recherchenort | Abschlußdatum der Recherche | Prüfer |
|---|---|---|
| Den Haag | 23. Februar 2024 | Steinmetz, Johannes |

EPO FORM 1503 03.82 (P04C03)

**EP 4 530 613 A1**

## ANHANG ZUM EUROPÄISCHEN RECHERCHENBERICHT
## ÜBER DIE EUROPÄISCHE PATENTANMELDUNG NR.

EP 23 19 9686

23-02-2024

| Im Recherchenbericht angeführtes Patentdokument | Datum der Veröffentlichung | Mitglied(er) der Patentfamilie | Datum der Veröffentlichung |
|---|---|---|---|
| US 2022167935 A1 | 02-06-2022 | EP 4014874 A1 | 22-06-2022 |
| | | JP 7373323 B2 | 02-11-2023 |
| | | JP 2021037031 A | 11-03-2021 |
| | | US 2022167935 A1 | 02-06-2022 |
| | | WO 2021044754 A1 | 11-03-2021 |
| CN 116392153 A | 07-07-2023 | KEINE | |
| US 2022366543 A1 | 17-11-2022 | US 2022366543 A1 | 17-11-2022 |
| | | WO 2021162026 A1 | 19-08-2021 |

EPO FORM P0461